# EUROPEAN PATENT SPECIFICATION

(11) **EP 0 345 777 B1**
(45) Date of publication and mention of the grant of the patent: **18.09.1996**
(21) Application number: 89110385.5
(22) Date of filing: 08.06.1989
(51) Int. Cl.: G01N 33/543

(54) **Immunoassay**
Immuntest
Immunoessai

(30) Priority: 10.06.1988 JP 143257/88
(43) Date of publication of application: 13.12.1989
(73) Proprietor: DAIICHI PURE CHEMICALS CO. LTD., Chuo-ku Tokyo (JP)
(72) Inventor: Nozawa, Masayuki, 5-5-12, Narihira Sumida-ku Tokyo (JP)
(74) Representative: Wächtershäuser, Günter, Prof. Dr.

(56) References cited:
- EP-A- 0 232 816
- EP-A- 0 308 704

## Description

### BACKGROUND OF THE INVENTION

### i) Field of the invention

This invention relates to a non-homogeneous immunoassay, and more specifically to a method for measuring an antigen or antibody in a sample solution without need for separation of a solid adsorbent and a liquid phase from each other subsequent to a non-homogeneous immunoreaction.

### ii) Description of the Background Art

As methods for immunologically analyzing components in various samples such as serum and urine by using the specificity of an antigen-antibody reaction, there are simple immunodiffusion methods in which an antigen-antibody complex is measured directly based on the amount of a precipitate, a change in turbidity, or the like; and immunoassays, such as radioimmunoassay (RIA), enzyme immunoassay (EIA) and fluorescent immunoassay (FIA), in which the amount of an antigen-antibody complex is determined by using a labelling substance such as a radioisotope, enzyme or pigment and measuring its activity or concentration in accordance with a method such as nephelometry. Compared with the former methods, the latter methods are superior in sensitivity quantitativeness, etc. The latter methods have hence been finding more utility in the field of analytical chemistry, especially, clinical analytical chemistry. Of these immunoassays using a labelling substance, RIA involves problems regarding the handling of an isotope and disposal of waste. There is thus a strong demand for the automation of immunoassays which use a labelling substance such as an enzyme or pigment.

EP-A-0 232 816 describes a heterogeneous specific binding assay employing an aggregatable binding reagent. After an immunoreaction with assay reagents including a labeled reagent, an immobilizable component is precipitated with a binding substance. Free and bound species of the labeled reagent are formed as a function of the amount of the analyte in the test medium. One of the free and bound species is immobilized and the immobilized labeled reagent is separated from labelled reagent which has not been immobilized. The amount of label in the liquid phase is determined and related to the amount of analyte in the test medium.

EP-A-0 308 704 which represents a state of the art pursuant to Art. 54(3) EPC describes a heterogeneous enzyme immunoassay which makes use of a dry-type chemical analytical film containing all reagents necessary for measuring the enzyme activity.

However, the automation of an immunoassay which uses a labelling substance, especially, the automation of a non-homogeneous immunoassay having excellent sensitivity and quantitativeness requires washing, centrifugation, etc. for the separation of a solid adsorbent and a liquid phase from each other (B/F separation). This has made the automation difficult. Non-homogeneous immunoassays using a labelling substance have been automated by means of a special apparatus designed exclusively therefor. However, this requires the installation of such an additional apparatus. Another problem has also come to the surface that the efficiency of the work cannot be improved so much by its use.

In the field of clinical chemical analyses, automatic analyzers generally called "biochemical autoanalyzers" have been finding wide-spread utility in recent years, whereby a substantial progress has been achieved in the automation of biochemical analyses. They are thus used a general-purpose apparatus in this field. Accordingly, it is also strongly desired to realize the automation of immunoassays by using a biochemical autoanalyzer, which has already been installed and used widely, without need for any special apparatus.

The present inventors hence measured liquid phases as samples by a general-purpose apparatus, using commercially-available non-homogeneous immunoassay kits which employed a solid adsorbent such as polystyrene beads or glass beads. However, their quantitativeness, accuracy and the like were too poor to use them as they were.

### SUMMARY OF THE INVENTION

With the foregoing in view, the present inventors have carried out an investigation with a view toward providing a solution to the above problems. A solid-liquid non-homogeneous immunoassay is generally conducted by measuring a labelled antigen or labelled antibody on a solid adsorbent subsequent to an immunoreaction. However, it has unexpectedly found that the above object can be attained by measuring the labelled antigen or labelled antibody in a liquid phase without need for the separation of the solid adsorbent and the liquid phase from each other subsequent to the reaction, leading to the completion of this invention.

Accordingly, the present invention provides a non-homogeneous immunoassay for measuring an antigen or antibody in a sample solution by reacting the antigen or antibody in the sample solution with a corresponding antibody or antigen immobilized on a solid adsorbent in the presence of a labeled antigen or antibody in a non-homogeneous system, characterized in that the labeled antigen or antibody in a liquid phase is measured after completion of the reaction without the need of separation of solid absorbent and liquid phase by an autoanalyzer that provides for selective removal of a portion of the liquid phase.

### BRIEF OF DESCRIPTION OF THE DRAWINGS

FIG. 1 diagrammatically illustrates a calibration curve for β₂m in Example 1; and
FIG. 2 diagrammatically depicts a calibration curve for AFP in Example 2.

### DETAILED DESCRIPTION OF THE INVENTION AND PREFERRED EMBODIMENTS

Solid matrices known per se in the art can be used as they are in the assay of this invention. Exemplary usable solid matrices include plastic beads such as polystyrene beads, latex particles, agarose gel, dextran gel, polyacrylamide gel, cellulose particles, ion-exchange resins, silicone, glass, microcapsules and metal colloids. It is also possible to use as a solid matrix a glass or plastic tube or cup which is employed as a reaction vessel. Of these, plastic beads and latex particles are particularly preferred. Holding of an antigen or antibody on a solid matrix can be effected by either physical adsorption or chemical covalent bonding, which is known per se in the art.

As labelling substances usable in the present invention, any labelling substances useful for general non-homogeneous immunoassays can be employed because the assay of this invention is an ordinary non-homogeneous immunoassay. Although the assay of this invention can of course be applied to RIA, RIA is accompanied by problems in the handling of a radio-isotope and the disposal of waste. As labelling susbtances, non-radioactive substances are thus more preferred.

As suitable labelling substances, may be mentioned enzymes, chromogens, chelating agents, fluorescent dyes, luminous substances and the like. For example, preferable enzymes include peroxidases, glucose oxidases, galactosidases, alkaline phosphatases, catalases and acetylcolinesterases.

Non-homogeneous immunoassays useful in the practice of this invention include, for example, competitive immunoassays such as the double antibody solid phase method, solid phase method and sandwich method.

Upon practice of the present invention, the amount of the labelling substance to be used is determined in accordance with the mole number of an antigen or antibody as the target of the assay and the range of the assay. In general, the labelling substance is used in a greater amount when the target antigen or antibody of the assay is contained in a large mole number. On the other hand, the amount of the labelling substance is reduced when the mole number of the target antigen or antibody of the assay is small. This can improve the quantitativeness further. If the labelling substance is used in a large excess relative to the amount of the target antigen or antibody of the assay, the sensitivity and accuracy of the assay are reduced. If the labelling substance is used in an unduly small amount on the other hand, the range of the assay is narrowed. Accordingly, where the target antigen or antibody of the assay reacts with the same equivalent amount of the labelling substance, it is desirable to use the labelling agent at a molar ratio of about 1-1,000 times relative to the target antigen or antibody of the assay.

After completion of the immunoreaction, the labelling substance in the liquid phase can be measured by simply subjecting the reaction mixture to a general autoanalyzer as is without separating the solid matrix from the reaction mixture.

Exemplary antigens or antibodies measurable by the present invention include known substances usually measured by a non-homogeneous immunoassay, for example, those listed in Ishikawa et al: "Koso Meneki Sokuteiho (Enzyme immunoassay)", Igakushoin (1987). Measurable antigens or antibodies are however not limited to them so long as a non-homogeneous immunoassay is feasible. Typical antibodies include β₂-microglobulin (β₂m), α-fetoprotein, carcinoembryonic antigen (CEA), ferritin, IgE, insulin, etc.

### EXAMPLES

The invention will next be described by the following examples.

### Example 1 : Measurement of β₂-microglobulin (β₂m)

### (1) Anti-human β₂m antibody-coated polystyrene beads:

Anti-human β₂m-monoclonal antibody (anti-human β₂m-McAb), which had been isolated in a purified form from ascitic fluid by ammonium sulfate fractionation and by ion-exchange using "DEAE Sephacryl" (trade name; product of Pharmacia AB), was dissolved at a concentration of 0.02 mg/ml in a 0.05 M carbonate buffer (pH 9.6) which contained 0.05 M of sodium chloride. Immersed in 50 ml of the resultant antibody solution was 20 g of polystyrene beads which had a diameter in a range of 0.2-0.3 mm and had been washed thoroughly. After allowing the mixture to stand overnight at 2-10°C, the antibody solution was removed and the thus-treated polystyrene beads were immersed in a 0.025 M phosphate buffer (pH 7.2) containing 0.1% of bovine serum albumin and 0.15 M of sodium chloride (hereinafter referred to as "BSA-PBS"). The mixture was then allowed to stand at least overnight at 2-4°C. The resulting polystyrene beads were washed thoroughly with PBS and then immersed in the same buffer to provide anti-human β₂m antibody-coated polystyrene beads.

### (II) Enzyme-labelled anti-human β₂m antibody:

Five milligrams of purified anti-human β₂m-McAb purified in a similar manner to the above procedure (I) and having a different dicernible antigen determinant were dissolved in 1 ml of a 0.1 M acetate buffer (pH 4.2), followed by addition of 0.25 mg of pepsin. The resultant mixture was allowed to stand overnight at 37°C under heat. After adding 0.1 ml of a 2 M tris-HCl buffer (pH 8.0) to terminate the reaction, the reaction mixture was subjected to gel chromatography on a column which had a diameter of 2.0 cm and a height of 60 cm and was packed with "ULTRO GEL AcA" (trade name; product of LKB Company) equilibrated in advance with a 0.1 M phosphate buffer (pH 6.5) containing 0.2 M of sodium chloride, thereby obtaining 2.5 mg of F(ab′)₂. Thereafter, the maleimide method proposed by Isikawa et al. in Scand. J. Immunol. 8, Suppl. 7: 43, 1978 was followed to obtain β-galactosidase-labelled anti-human β₂m-McAb F(ab').

### (III) Assay:

### (a) Enzyme-labelled antibody solution:

β-Galactosidase-labelled anti-human β₂m-McAb obtained in the above procedure (II) was diluted with 0.5% BSA-PBS to give an absorbance of 0.0025 OD as measured at 280 nm (absorbance of the enzyme-labelled antibody alone without BSA).

### (b) Standard solution:

Purified human β₂m was diluted with horse serum to 500 ng/ml, 250 ng/ml, 100 ng/ml, 50 ng/ml, 25-ng/ml and 12.5 ng/ml concentrations.

### (c) Immunoreaction reagent-reaction solution:

An immunoreaction reagent-reaction solution mixture was prepared by adding the anti-human β₂m antibody-coated polystyrene beads and 0.3 ml of the enzyme-labelled antibody solution to a sample cup for autoanalyzer.

### (d) Analyzer:

Hitachi 7050 Model was used.

### (e) Operation:

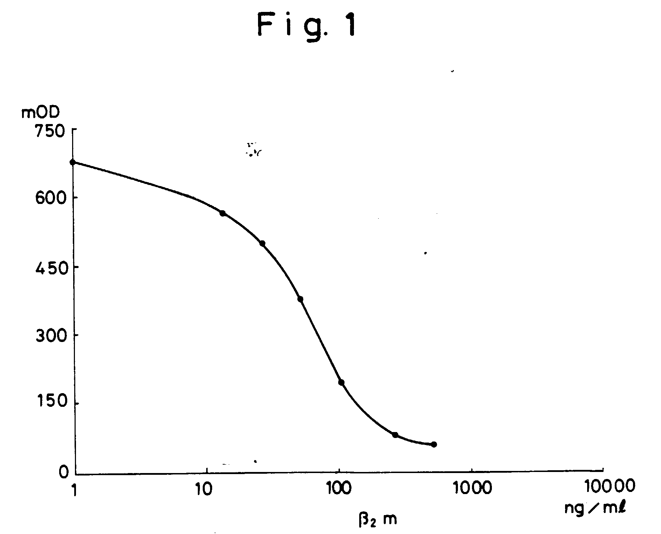

Fifty microliters of the standard solution or sample were added to the mixture (c), followed by through mixing. The resultant immunoreaction mixture was left over for 1 hour at room temperature and the reaction vessel was placed on a sample rack of the autoanalyzer. The autoanalyzer was provided with 0.1 M PBS (pH 7.0) containing 10 mM of o-nitrophenyl β-D-galactopyranoside as a substrate. Using the liquid phase as a sample, 20 µl of the sample was reacted with 350 µl of the substrate. A change in absorbance was measured at 415 nm. The concentration o β₂m in the sample was determined on the basis of the measurement data of the standard solution. A calibration curve thus obtained is shown in FIG. 1.

### Example 2 : Assay of α-fetoprotein (AFP)

### (I) Anti-human AFP antibody-coated polystyrene latex:

After mixing 5 ml of a 1% suspension of 1.0 µm polystyrene latex (product of Sekisui Chemical Co., Ltd.) in a 0.05 M glycine buffer (pH 8.5) with 5 ml of a 0-.5 mg/ml anti-AFPMcAbIgG fraction, the mixture was left over for 2 hours at room temperature. Five milliliters of the same buffer containing 0.5% of BSA were added and the resultant mixture was allowed to stand overnight at 2-10°C. Unsensitized IgG was then washed and removed by centrifugation. The residue was resuspended in a 0.025 M phosphate buffer(pH 7.2) containing 0.15 M of sodium chloride and then stored at 4°C or lower.

### (II) Enzyme-labelled antibody:

Using peroxidase (horse raddish) as an enzyme, an enzyme-labelled antibody was prepared in the following manner by the method proposed by Nakane et al. in J. Histochem Cylochem., 22, 1084 (1974).

Four milligrams of peroxidase (product of Toyobo Co., Ltd.) was dissolved in 1 ml of water, followed by addition of 0.2 ml of a 0.1 M NaIO₄ solution. They were reacted for 20 minutes at room temperature. After completion of the reaction, the reaction mixture was immersed in a 1 mM sodium acetate buffer. Subsequent to an adjustment to pH 9-9.5 with a 0.2 M sodium carbonate buffer, 5 mg of anti-human AFPMcAb-Fab' was added, followed by a reaction for 2 hours at room temperature.

Added next was 0.1 ml of a 4 mg/ml of NaBH₄ solution. After allowing the resultant mixture to stand at 4°C for 2 hours, the reaction mixture was subjected to gel chromatography on "ULTRO GEL AcA 44" (trade name; product of LKB Company). Based on the absorbances at 280 nm and 405 nm peak fractions of the enzyme-labelled antibody were collected and the enzyme labelled antibody was stored in the concentrated form.

### (III) Measurement:

### (a) Enzyme-labelled antibody solution:

Peroxidase-labelled anti-human AFPMcAb obtained in the above procedure (II) was diluted with 0.1% BSA-PBS to give an absorbance of 0.000125 OD as measured at 280 nm (absorbance of the enzyme-labelled antibody alone without BSA).

### (b) Standard solution:

Purified human AFP was diluted with goat serum to give 800 ng/ml, 400 ng/ml 200 ng/ml, 100 ng/ml, 50 ng/ml, 25 ng/ml and 12.5 ng/ml concentrations, separately.

### (c) Immunoreaction reagent-reaction solution:

An immunoreaction reagent-reaction solution mixture was prepared by adding the anti-human AFP antibody-coated polystyrene latex and 0.5 ml of the enzyme-labelled antibody solution to a sample cup for autoanalyzer.

### (d) Analyzer:

Hitachi 7050 Model was used.

### (e) Operation:

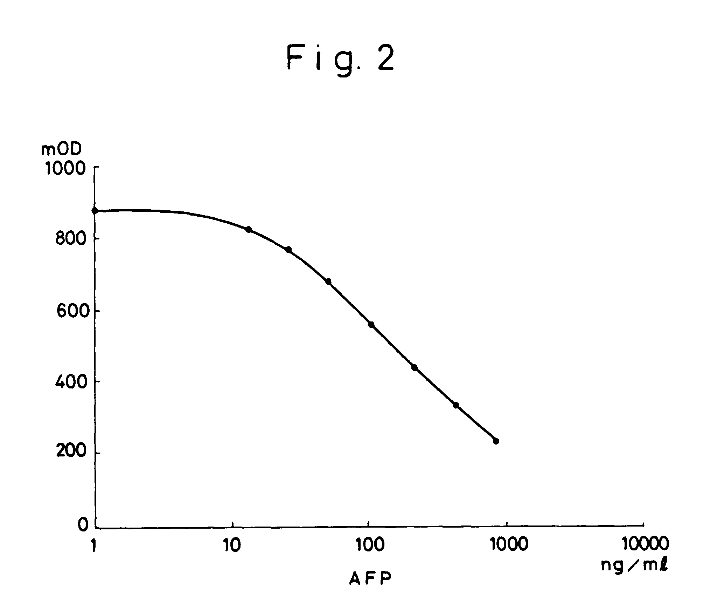

Fifty microliters of the standard solution or sample were added to the mixture (c), followed by through mixing. The resultant immunoreaction mixture was left over for 1 hour at room temperature and the reaction vessel was placed on a sample rack of the autoanalyzer. The autoanalyzer was provided with a 0.1 M phosphate-citrate buffer (pH 5.0) containing 10 mM of o-phenylenediamine and 0.01% of hydrogen peroxide. Using the liquid phase of the immunoreaction mixture as a sample, 20 µl of the sample was reacted with 350 µl of the substrate. A change in absorbance was measured at 450 nm. The concentration of AFP in the sample was determined on the basis of the measurement data of the standard solution in a similar manner to Example 1. A calibration curve thus obtained is shown in FIG. 2.

According to the present invention, non-homogeneous immunoassays, whose automation have been difficult because of the need for washing and centrifugation, can be easily automated by subjecting immunoreacted mixtures as samples to a biochemical autoanalyzer without need for special apparatus.

## Claims

1. A non-homogeneous immunoassay for measuring an antigen or antibody in a sample solution by reacting tine antigen or antibody in the sample solution with a corresponding antibody cr antigen immobilized on a solid adsorbent in the presence of a labelled antigen or antibody in a non-homogeneous system, characterized in that the labelled antigen or antibody in a liquid phase is measured after completion of the reaction without the need of separation of solid absorbent and liquid phase by an autoanalyzer that provides for selective removal of a portion of the liquid phase.

2. The immunoassay as claimed in Claim 1, wherein the labelled antigen or antibody is added at a molar ratio of 1-1,000 relative to the antigen or antibody in the sample solution to be measured.

## Patentansprüche

1. Nicht-homogener Immunoassay zum Messen eines Antigens oder Antikörpers in einer Probenlösung durch Umsetzen des Antigens oder Antikörpers in der Probenlösung mit einem entsprechenden, auf einem festen Adsorptionsmittel immobilisierten Antikörper oder Antigen in Gegenwart eines markierten Antigens oder Antikörpers in einem nicht-homogenen System, dadurch gekennzeichnet, daß das markierte Antigen oder der markierte Antikörper in einer flüssigen Phase, nach Abschluß der Reaktion, mittels eines Autoanalysators gemnessen wird, der für eine selektive Entfernung eines Teiles der flüssigen Phase sorgt, ohne daß die Trennung des festen Absorptionsmittels und der flüssigen Phase erforderlich ist.

2. Immunoassay nach Anspruch 1, worin das markierte Antigen oder der markierte Antikörper in einem molaren Verhältnis von 1-1.000 mit Bezug auf das Antigen oder den Antikörper in der zu messenden Probenlösung hinzugegeben wird.

## Revendications

1. Immunoessai non-homogène pour l'analyse d'un antigène ou d'un anticorps, dans une solution d'échantillon par réaction de l'antigène ou de l'anticorps de la solution d'échantillon, avec un anticorps ou un antigène, correspondant immobilisé sur un adsorbant solide en présence d'un antigène marqué ou d'un anticorps marqué dans un système non-homogène, caractérisé en ce qu'on analyse l'antigène marqué ou l'anticorps marqué, dans une phase liquide après l'achèvement de la réaction sans nécessité de recourir à une séparation de l'adsorbant solide et de la phase liquide, au moyen d'un auto-analyseur qui assure l'élimination sélective d'une partie de la phase liquide.

2. Imunoessai selon la revendication 1, dans lequel on ajoute l'antigène marqué ou l'anticorps marqué, dans un rapport molaire de 1-1000 par rapport à l'antigène ou l'anticorps, dans la solution d'échantillon à analyser.
